(19) Europäisches Patentamt
European Patent Office
Office européen des brevets

(11) Numéro de publication : **0 457 687 B1**

# (12) FASCICULE DE BREVET EUROPEEN

(45) Date de publication du fascicule du brevet :
**15.12.93 Bulletin 93/50**

(51) Int. Cl.$^5$ : **A61K 7/42**

(21) Numéro de dépôt : **91401275.2**

(22) Date de dépôt : **17.05.91**

## (54) Emulsion cosmétique filtrante comprenant un filtre UV-A et un filtre UV-B.

(30) Priorité : **18.05.90 FR 9006278**

(43) Date de publication de la demande :
**21.11.91 Bulletin 91/47**

(45) Mention de la délivrance du brevet :
**15.12.93 Bulletin 93/50**

(84) Etats contractants désignés :
**AT BE CH DE DK ES FR GB GR IT LI NL SE**

(56) Documents cités :
**EP-A- 0 087 098**
**EP-A- 0 365 370**
**GB-A- 2 121 801**

(73) Titulaire : **L'OREAL**
**14, Rue Royale**
**F-75008 Paris (FR)**

(72) Inventeur : **Forestier, Serge**
**16 Allée Ferdinand Buisson**
**F-77410 Claye-Souilly (FR)**
Inventeur : **Eteve, Martine**
**19, rue Charles Fournier**
**F-75013 Paris (FR)**

(74) Mandataire : **Casalonga, Axel et al**
**BUREAU D.A. CASALONGA - JOSSE**
**Morassistrasse 8**
**D-80469 München (DE)**

EP 0 457 687 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition cosmétique filtrant les radiations ultraviolettes, sous forme d'émulsion, comprenant en association un filtre UV-A hydrophile et un filtre UV-B lipophile sélectionnés de façon à accroître l'indice de protection de ladite composition, ainsi que l'utilisation de ladite composition pour protéger l'épiderme humain contre les radiations ultraviolettes.

On sait que les radiations lumineuses de longueurs d'onde comprises entre 280 et 400 nm permettent le brunissement de l'épiderme humain et que les rayons de longueurs d'onde comprises entre 280 et 320 nm connus sous la dénomination UV-B provoquent également des érythèmes et des brûlures cutanées qui peuvent nuire au développement du bronzage.

Toutefois, si les rayons UV-B de longueurs d'onde comprises entre 280 et 320 nm jouent un rôle prépondérant dans la production de l'érythème solaire et doivent être filtrés, il n'en reste pas moins vrai que les rayons UV-A de longueurs d'onde comprises entre 320 et 400 nm provoquant le brunissement de la peau, sont susceptibles d'induire également une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photoallergiques.

Il peut donc être avantageux de filtrer l'ensemble du rayonnement de longueurs d'onde comprises entre 280 et 400 nm.

Ainsi, on a déjà proposé l'utilisation de composés actifs absorbant fortement les rayons UV sur une large bande dont, plus particulièrement, des dérivés du benzène [di(méthylidène camphre)] sulfonés sur le radical méthyle en position 10 du camphre tels que décrits dans le brevet français n° 2 528 420 de la demanderesse. Ces filtres absorbent fortement les rayons UV de longueurs d'onde comprises entre 280 et 400 nm avec des maxima d'absorption compris entre 320 et 400 nm pour les composés ayant des radicaux méthylidène camphre se trouvant en position para par rapport au noyau benzénique, et notamment aux alentours de 345 nm.

L'efficacité des compositions cosmétiques renfermant ces filtres UV à large spectre, exprimée par le facteur de protection solaire que l'on convient d'appeler "indice de protection ou IP", est bonne, mais s'avère encore insuffisante pour des peaux très sensibles ou continuellement exposées au rayonnement solaire, notamment en ce qui concerne l'indice de protection UV-B.

L'indice de protection ou IP peut s'exprimer par le rapport du temps d'irradiation nécessaire pour atteindre le seuil érythématogène avec le filtre UV au temps d'irradiation nécessaire pour atteindre le seuil érythématogène sans filtre UV.

On a également proposé l'utilisation de dérivés de triazine tels que ceux décrits dans le brevet américain US 4 724 137 et plus particulièrement la 2,4,6-tris[p-(2'-éthylhexyl-1'-oxycarbonyl)anilino]-1,3,5-triazine. Ces filtres absorbent fortement les rayons UV de longueurs d'onde comprises entre 280 nm et 320 nm avec un maximum d'absorption à 312 nm.

Cependant, l'indice de protection des compositions cosmétiques renfermant ce dernier filtre est encore, lui aussi, insuffisant dans le cas de peaux extrêmement sensibles.

Par ailleurs, sur le plan industriel, il est bien sûr avantageux de disposer de filtres UV qui permettent, à des concentrations faibles, d'obtenir des compositions solaires à indice de protection élevé.

La demanderesse vient de découvrir qu'en associant dans une émulsion, le dérivé du benzène [di(méthylidènecamphre)] sulfoné sur le radical méthyle en position 10 du camphre constitué par l'acide benzène 1,4-[di(3-méthylidène camphométhyl sulfonique)] ou ses sels solubles ou dispersibles en milieu aqueux, qui est un filtre UV-A ayant un maximum d'absorption aux alentours de 345 nm, avec la 2,4,6-tris-[p-(2'-éthylhexyl-1'-oxycarbonyl)anilino]-1,3,5-triazine, qui est un filtre UV-B ayant un maximum d'absorption à 312 nm, on obtenait, de façon surprenante, pour une émulsion renfermant une concentration donnée des deux filtres précités pris en combinaison, un indice de protection, notamment UV-B, largement supérieur aux indices de protection d'émulsions renfermant l'un ou l'autre de ces deux filtres à la même concentration et dans le même support.

La présente invention a donc pour objet une émulsion cosmétique filtrant les radiations ultraviolettes de longueurs d'onde comprises entre 280 et 400 nm renfermant, dans la phase aqueuse, l'acide benzène 1,4-[di(3-méthylidène camphométhyl sulfonique)], partiellement ou totalement neutralisé de formule (I) ci-dessous, en combinaison avec la 2,4,6-tris[p-(2'-éthylhexyl-1'-oxycarbonyl)-anilino]-1,3,5-triazine, dans la phase huileuse.

L'acide benzène 1,4-[di(3-méthylidène camphométhyl-sulfonique)], partiellement ou totalement neutralisé, a pour formule :

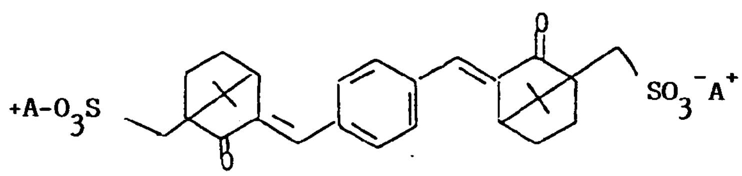

(I)

dans laquelle A désigne un atome d'hydrogène, un métal alcalin, un groupement $N(R)_4^+$, R désignant un atome d'hydrogène ou un radical alkyle ou hydroxyalkyle en $C_1$-$C_4$, ou encore un groupement $\frac{M^{n+}}{n}$ où $M^{n+}$ est un cation métallique polyvalent dans lequel n est égal à 2 ou 3 ou 4, $M^{n+}$ désignant de préférence $Ca^{2+}$, $Zn^{2+}$, $Mg^{2+}$, $Ba^{2+}$, $Al^{3+}$ ou $Zr^{4+}$.

Il est bien entendu que le composé de formule (I) peut donner lieu à l'isomère "cis-trans" autour d'une ou plusieurs double(s) liaison(s) et que tous les isomères font partie de l'invention.

La présente invention a également pour objet un procédé de traitement cosmétique de l'épiderme humain contre les radiations ultraviolettes de longueurs d'onde comprises entre 280 et 400 nm, consistant à appliquer sur la peau une quantité efficace de la composition cosmétique filtrante précitée sous forme d'émulsion comprenant en association l'acide benzène 1,4-[di(3-méthylidène camphométhyl sulfonique)], partiellement ou totalement neutralisé, et la 2,4,6,-tris[p-(2'-éthylhexyl-1'-oxycarbonyl)anilino]-1,3,5-triazine.

La composition cosmétique filtrante selon l'invention présente, outre son indice de protection élevé, l'avantage d'être thermiquement et photochimiquement stable, de n'être pas toxique et de présenter une parfaite innocuité vis-à-vis de la peau.

De plus, cette composition cosmétique ne colle pas au toucher, ne laisse pas subsister sur la peau, après application, de pellicule blanchâtre et présente également une grande persistance.

La persistance peut être définie comme la stabilité d'indice de protection de la composition filtrante au cours de l'exposition au soleil. Il est important que cette persistance soit élevée car il est nécessaire que l'indice de protection soit constant au cours de l'exposition, ce qui permet d'éviter des applications répétées à intervalles réguliers et rapprochés souvent nécessaires pour obtenir une protection efficace de la peau contre les rayons UV.

Cet indice de protection peut varier soit parce que le filtre est instable photochimiquement, soit parce qu'il pénètre dans la peau et ne joue plus son rôle, ou encore parce qu'il s'élimine au cours de la baignade.

La 2,4,6-tris[p-(2'-éthylhexyl-1'-oxycarbonyl)anilino]-1,3,5-triazine est vendue par la société BASF sous la dénomination commerciale UVINUL T 150.

Pour neutraliser l'acide benzène 1,4-[di(3-méthylidène camphométhyl sulfonique)], on peut utiliser les hydroxydes alcalins et plus particulièrement de sodium ou de potassium, l'ammoniaque, les alcanolamines, la triéthanolamine étant préférée. On obtient ainsi des sels de métaux alcalins, d'ammonium ou d'amines solubles dans l'eau.

Pour obtenir des sels de métaux polyvalents insolubles dans l'eau, de formule (I) dans laquelle A désigne un cation métallique polyvalent $M^{n+}$ de valence 2, 3 ou 4, on ajoute un sel ou un hydroxyde de métal polyvalent correspondant, éventuellement en solution ou en suspension aqueuse, à une solution aqueuse d'un composé de formule (I) dans laquelle A désigne un atome d'hydrogène, un métal alcalin tel que le sodium ou le potassium ou un reste ammonium.

Le sel de métal polyvalent peut être par exemple un halogénure, en particulier un bromure ou un chlorure, un nitrate, un acétate, un carbonate ou un sulfate.

L'addition du sel de métal polyvalent est effectuée sous agitation. Lorsque le composé de formule (I) est sous forme d'acide sulfonique, le pH du mélange réactionnel peut éventuellement être ajusté au voisinage de la neutralité en cours de réaction par addition d'une solution aqueuse d'un hydroxyde de métal alcalin ou d'ammonium.

Cette réaction peut également être réalisée en inversant l'ordre d'introduction des réactifs.

Le sel de métal polyvalent est de préférence utilisé en quantité stoechiométrique pour salifier le composé de formule (I) de départ.

Le composé de formule (I) dans laquelle A désigne un cation métallique polyvalent $M^{n+}$, précipite au cours de la réaction. Il est isolé par filtration, puis lavé à l'eau pour éliminer les sels minéraux.

Le composé de formule (I) sous forme d'acide ou de sel de métal alcalin, d'ammonium ou d'amine peut être préparé selon les modes opératoires décrits dans le brevet FR-2 528 420.

A titre de composés (I) dans lesquels A désigne $\frac{M^{n+}}{n}$ particulièrement préférés, on peut citer les composés

dans lesquels $M^{n+}$ désigne $Ca^{2+}$, $Zn^{2+}$, $Mg^{2+}$, $Al^{3+}$ ou $Zr^{4+}$.

Les émulsions solaires selon l'invention renferment, dans la phase aqueuse, l'acide benzène 1,4-[di(3-méthylidène camphométhyl sulfonique)], partiellement ou totalement neutralisé, qui est un composé hydrophile; il est utilisé dans des proportions, calculées sur la base de l'acide, comprises entre 0,1 et 10% en poids, et de préférence entre 1 et 6% par rapport au poids total de la composition.

Les émulsions solaires selon l'invention renferment, dans la phase huileuse, l'UVINUL T 150, qui est un composé lipophile, dans des proportions comprises entre 0,1 et 10% en poids, et de préférence entre 1 et 6% par rapport au poids total de la composition.

Dans le cas d'émulsions H/E, le pH est compris entre 4 et 9 et de préférence entre 5,5 et 8. Il peut être ajusté à l'aide d'un agent alcalinisant ou acidifiant usuel.

La phase huileuse est constituée de corps gras tels que :

- des huiles minérales comme l'huile de vaseline;
- des huiles végétales ou animales modifiées ou non telles que l'huile d'amande douce, l'huile d'avocat, l'huile de calophyllum, l'huile de ricin, l'huile d'olive, la lanoline et ses dérivés, le perhydrosqualène, l'huile d'arachide, l'huile de germes de blé, l'huile de lin, l'huile de jojoba, l'huile de noyaux d'abricots, l'huile de noix, l'huile de palme, l'huile de pistache, l'huile de sésame, l'huile de colza, l'huile de cade, l'huile de germes de maïs, l'huile de noyaux de pêches, l'huile d'oeillette, l'huile de pin, l'huile de soja, l'huile de carthame, l'huile de coprah, l'huile de noisette, l'huile de pépins de raisin ou encore l'huile de tournesol;
- des esters d'acides gras saturés ou insaturés ou des huiles de synthèse comme le palmitate d'isopropyle ou d'éthyle, l'adipate de diisopropyle, les myristates d'alkyle tels que ceux d'isopropyle, de butyle et de cétyle, le stéarate d'hexyle, le ricinoléate de cétyle, l'octanoate de stéaryle(huile de purcellin), l'éthyl-2 hexanoate de cétostéaryle, le malate de dioctyle, les esters d'acides gras en $C_8$-$C_{18}$ et de polyols tels que les esters de ces acides et du glycérol, par exemple les triglycérides de ces acides gras, ainsi que le polyisobutène.

La phase huileuse de ces émulsions selon l'invention peut également contenir certaines cires et notamment de la cire de Carnauba, de la cire d'abeille, de l'ozokérite, de la cire de Candelilla et des cires microcristallines, des huiles de silicone, ou encore des acides gras et des alcools gras en $C_{12}$-$C_{18}$.

La phase aqueuse des émulsions selon l'invention peut également contenir des monalcools ou polyalcools inférieurs contenant 1 à 6 atomes de carbone. Les monoalcools ou polyalcools particulièrement préférés sont l'éthanol, l'isopropanol, le propylèneglycol et la glycérine.

Les émulsionnants sont présents soit dans la phase grasse soit dans la phase aqueuse ou dans les deux phases à la fois.

La proportion des émulsionnants est généralement comprise entre 1 et 15% par rapport au poids total de l'émulsion.

Ces émulsionnants sont choisis parmi :

- les alkyl ($C_8$-$C_{12}$) phénols polyoxyéthylénés contenant 9 à 15 moles d'oxyde d'éthylène;
- les alcools gras en $C_{12}$-$C_{18}$ polyoxyéthylénés comportant au moins 4 et de préférence 4 à 35 moles d'oxyde d'éthylène;
- les alcools gras en $C_{10}$-$C_{18}$ polyglycérolés comportant 4 à 10 motifs glycérol;
- les alcools gras en $C_{12}$-$C_{18}$ polyoxypropylénés comportant de préférence 3 moles d'oxyde de propylène;
- les esters d'acides gras en $C_{12}$-$C_{18}$ à la fois polyoxyéthylénés et polyglycérolés ou monoglycérolés;
- les esters d'acides gras en $C_{12}$-$C_{18}$ du sorbitan, polyoxyéthylénés, contenant 10 à 20 moles d'oxyde d'éthylène;
- les esters d'acides gras en $C_{12}$-$C_{18}$ polyoxyéthylénés comportant au moins 2 motifs d'oxyde d'éthylène et de préférence 4 à 20 motifs d'oxyde d'éthylène;
- l'huile de ricin polyoxyéthylénée contenant 10 à 60 moles d'oxyde d'éthylène;
- les copolymères oxyde de propylène/oxyde d'éthylène;
- la lécithine de soja ou de jaune d'oeuf;
- les sucroglycerides;
- les savons;
- les esters phosphoriques d'alcools gras;
- les sulfates d'alcools gras éventuellement oxyéthylénés;
- les alcools de lanoline polyoxyéthylénés et comportant au moins 4 motifs d'oxyde d'éthylène.

Les émulsions selon l'invention peuvent également contenir des épaississants, des adoucissants, des humectants, des tensio-actifs, des conservateurs, des anti-mousses, des parfums, des colorants ou pigments ayant pour fonction de colorer la composition elle-même ou la peau ou d'absorber ou de réfléchir les radiations solaires, ou tout autre ingrédient habituellement utilisé en cosmétique.

Ces émulsions peuvent se présenter sous différentes formes telles qu'un lait, une crème, et être conditionnées en aerosol.

Ces émulsions peuvent éventuellement renfermer, outre l'association des deux filtres selon l'invention, d'autres filtres UV hydrosolubles ou liposolubles bien connus tels que, à titre d'exemple, l'huile de café, les dérivés de l'acide salicylique, les dérivés de l'acide cinnamique, les esters et dérivés de l'acide p-amino benzoïque, les anthranilates, les dérivés de benzophénone, les dérivés du benzylidène camphre tels que le p-méthylbenzylidène camphre, les dérivés du dibenzoylméthane, les dérivés du benzotriazole, les dérivés du benzoxazole, les dérivés du benzimidazole et les α-cyano β,β-diphényl acrylates d'alkyle ($C_2$-$C_{10}$).

L'invention sera mieux comprise à l'aide des exemples suivants.

EXEMPLES DE PREPARATION

EXEMPLE 1

Préparation d'un composé de formule générale (I) dans laquelle A désigne $\frac{M^{n+}}{n}$, $M^{n+}$ représentant $Mg^{2+}$

On ajoute 3 litres d'eau à 1 kg de solution aqueuse à 30% d'acide téréphtalylidène dicamphosulfonique (0,533 mole). On introduit, sous agitation, 108 g (0,533 mole) de chlorure de magnésium hexahydraté et on maintient l'agitation pendant 90 minutes.

On filtre le mélange réactionnel. Le solide blanc obtenu est lavé à l'eau sous agitation, puis à l'éthanol et séché sous pression réduite.

On obtient ainsi 260 g de produit attendu sous forme d'une poudre blanche possédant les caractéristiques suivantes :

Point de fusion : > 300°C

Analyse élémentaire : $C_{28}H_{32}O_8S_2Mg$, $5H_2O$

| | C% | H% | O% | S % | Mg% |
|---|---|---|---|---|---|
| Calculé : | 49,81 | 6,27 | 30,81 | 9,49 | 3,60 |
| Trouvé : | 51,14 | 6,57 | 29,51 | 8,67 | 4,11 |

EXEMPLE 2

Préparation d'un composé de formule générale (I) dans laquelle A désigne $\frac{M^{n+}}{n}$, $M^{n+}$ représentant $Zn^{2+}$

On ajoute 700 $cm^3$ d'eau à 200 g de solution aqueuse à 29,5% d'acide téréphtalylidène dicamphosulfonique (0,105 mole). On introduit, sous agitation, 14,2 g (0,105 mole) de chlorure de zinc dihydraté en solution dans 160 $cm^3$ d'eau et on maintient l'agitation pendant 3 heures.

On filtre le mélange réactionnel. Le solide blanc est lavé à l'eau sous agitation et séché sous pression réduite.

On obtient ainsi 57 g de produit attendu sous forme d'une poudre blanche possédant les caractéristiques suivantes :

Point de fusion : >300°C

Analyse élémentaire : $C_{28}H_{32}O_8S_2Zn$, $6H_2O$

| | C% | H% | O% | S% | Zn% |
|---|---|---|---|---|---|
| Calculé : | 45,78 | 5,99 | 30,52 | 8,72 | 8,86 |
| Trouvé : | 45,30 | 6,03 | 31,09 | 8,68 | 9,11 |

EXEMPLE 3

Préparation d'un composé de formule générale (I) dans laquelle A désigne $\frac{M^{n+}}{n}$, $M^{n+}$ représentant $Al^{3+}$

On ajoute 30 $cm^3$ d'eau à 30 g de solution aqueuse à 29,5% d'acide téréphtalylidène dicamphosulfonique (0,016 mole). On introduit, sous agitation, 2,41 g (0,013 mole) de chlorure d'aluminium trihydraté en solution

dans 10 $cm^3$ d'eau. On ajoute 50 $cm^3$ d'eau et on maintient l'agitation pendant 3 heures.

On filtre le mélange réactionnel. Le solide blanc obtenu est lavé à l'eau sous agitation et séché sous pression réduite.

On obtient ainsi 8,4 g de produit attendu sous forme d'une poudre blanche possédant les caractéristiques suivantes :

Point de fusion : > 300°C

Analyse élémentaire : $C_{84}H_{96}O_{24}S_6Al_2$, $22H_2O$

| | C% | H% | O% | S% | Al% |
|---|---|---|---|---|---|
| Calculé : | 47,28 | 6,57 | 34,50 | 9,00 | 2,53 |
| Trouvé : | 46,71 | 6,46 | 31,96 | 8,84 | 2,43 |

EXEMPLE 4

Préparation d'un composé de formule générale (I) dans laquelle A désigne $\frac{M^{n+}}{n}$, $M^{n+}$ représentant $Ca^{2+}$

On ajoute 100 $cm^3$ d'eau à 100 g de solution aqueuse à 29,5% d'acide téréphtalylidène dicamphosulfonique (0,0525 mole). On introduit, sous agitation, 7,7 g (0,0525 mole) de chlorure de calcium dihydraté en solution dans 50 $cm^3$ d'eau et on maintient l'agitation pendant 1 heure.

On filtre le mélange réactionnel. Le solide blanc obtenu est lavé à l'eau sous agitation et séché sous pression réduite.

On obtient ainsi 27 g de produit attendu sous forme d'une poudre blanche possédant les caractéristiques suivantes :

Point de fusion : >300°C

Analyse élémentaire : $C_{28}H_{32}O_8S_2Ca$, $4H_2O$

| | C% | H% | O% | S% | Ca% |
|---|---|---|---|---|---|
| Calculé : | 49,92 | 5,94 | 28,53 | 9,50 | 5,94 |
| Trouvé : | 49,61 | 5,99 | 29,06 | 8,90 | 6,44 |

EXEMPLE 5

Préparation d'un composé de formule générale (I) dans laquelle A désigne $\frac{M^{n+}}{n}$, $M^{n+}$ représentant $Zr^{4+}$

On ajoute 750 $cm^3$ d'eau à 200 g de solution aqueuse à 29,5% d'acide téréphtalylidène dicamphosulfonique (0,106 mole).

On introduit, sous agitation, 12,3 g (0,053 mole) de chlorure de zirconium anhydre dissous dans 925 $cm^3$ d'eau et on maintient l'agitation pendant 1 heure.

On filtre le mélange réactionnel. Le solide blanc obtenu est lavé à l'eau sous agitation et séché sous pression réduite.

On obtient 46,1 g de produit sous forme d'une poudre blanche présentant les caractéristiques suivantes :

Point de fusion : > 250°C

Rapport en poids entre le carbone et le soufre pour :

$C_{56}H_{64}O_{16}S_4Zr$

Calculé : 5,25

Trouvé : 5,20

EXEMPLES DE FORMULATION

EXEMPLE A

On prépare la composition solaire suivante :

A - Phase grasse

| | |
|---|---|
| - Lanoline | 7,0 g |
| - Ester d'acide oléique polyéthoxylé et de glycérol vendu sous la dénomination "LABRAFIL M 1969 CS" par la Société GATTEFOSSE | 3,0 g |
| - Mélange de stéarate de glycéryle et de stéarate de polyéthylèneglycol 100 vendu sous la dénomination "ARLACEL 165" par la société ICI | 5,0 g |
| - Huile de silicone | 0,8 g |
| - Alcool cétylique | 3,0 g |
| - Acide stéarique | 2,5 g |
| - Alcool myristique polyoxypropyléné à 3 moles d'oxyde de propylène vendu sous la dénomination "WITCONOL APM" par la société WITCO | 7,5 g |
| - Malate de dioctyle vendu sous la dénomination "CERAPHYL 45" par la société VAN DICK | 7,5 g |
| - Triéthanolamine | 0,2 g |
| - Hexadécylphosphate de potassium vendu sous la dénomination "AMPHISOL K" par la Société GIVAUDAN | 0,5 g |
| - 2,4,6-tris[p-(2'-éthylhexyl-1'-oxycarbonyl)anilino] 1,3,5-triazine vendue par la société BASF sous la dénomination "UVINUL T 150" | 3,0 g |
| - Conservateur, antioxydant | qs |
| TOTAL : | 40,2 g |

B - Phase aqueuse

| | |
|---|---|
| - Acide benzène 1,4[di(3-méthylidène camphométhyl sulfonique)] | 3,0 g MA |
| - Triéthanolamine | 1,8 g |
| - Parfum Conservateur | qs |
| - Eau | 54,2 g |

On réalise l'émulsion en additionnant la phase grasse portée aux environs de 80°C à la phase aqueuse portée à la même température et sous agitation rapide. On obtient une émulsion huile-dans-l'eau se présentant sous la forme d'une crème.

## EXEMPLE B

On prépare la composition solaire suivante :

A - Phase grasse :

| | |
|---|---|
| - Alcool cétylstéarylique | 5,6 g |
| - Alcool cétylstéarylique polyoxyéthylèné à 33 moles d'oxyde d'éthylène | 1,4 g |
| - Mélange de monostéarate et de distéarate de glycéryle non autoémulsionnable | 2,0 g |
| - Huile de silicone | 1,5 g |
| - Alcool cétylique | 1,5 g |
| - Alcool myristique polyoxypropylèné à 3 moles d'oxyde de propylène vendu sous la dénomination "WITCONOL" APM par la société WITCO | 7,5 g |
| - Malate de dioctyle vendu sous la dénomination "CERAPHYL 45" par la société VAN DICK | 7,5 g |
| - 2,4,6-tris[p-(2'éthylhexyl-1'-oxycarbonyl)anilino] - 1,3,5-triazine vendue par la société BASF sous la dénomination "UVINUL T 150" | 3,0 g |
| - Conservateur qs | |
| TOTAL : | 30,2 g |

B - Phase aqueuse :

| | |
|---|---|
| - Acide benzène 1,4-[di(3-méthylidène camphométhyl sulfonique] | 3,0 g MA |
| - Glycérine | 20,0 g |
| - Triéthanolamine | 1,8 g |
| - Parfum, conservateur qs. | |
| - Eau | 44,2 g |

On réalise l'émulsion en additionnant la phase grasse portée aux environs de 80°C à la phase aqueuse portée à la même température et sous agitation rapide. On obtient une émulsion huile-dans-l'eau se présentant sous la forme d'une crème.

EXEMPLE C

On prépare la composition solaire suivante de la même façon qu'à l'exemple A :

A Phase grasse

| | |
|---|---|
| - Alcool cétylstéarylique | 5,6 g |
| - Alcool cétylstéarylique polyoxyéthyléné à 33 moles d'oxyde d'éthylène | 1,4 g |
| - Mélange de monostéarate et de distéarate de glycéryle non autoémulsionnable | 2,0 g |
| - Huile de silicone | 1,5 g |
| - Alcool cétylique | 1,5 g |
| - Malate de dioctyle vendu sous la dénomination "CERAPHYL 45" par la société VAN DYCK | 15,0 g |
| - 2,4,6-tris[p-(2'-éthylhexyl-1'-oxycarbonyl)anilino] 1,3,5-triazine vendue par la société BASF sous la dénomination "UVINUL T 150" | 5,0 g |
| - Glycérine | 20,0 g |
| - Conservateur, antioxydant | qs |
| TOTAL : | 52,2 g |

B - Phase aqueuse

| | |
|---|---|
| - Acide benzène 1,4[di(3-méthylidène camphométhyl sulfonique)] | 1,0 g MA |
| - Triéthanolamine | 0,6 g |
| - Parfum, conservateur | qs |
| - Eau | 45,4 g |

EXEMPLE D

On prépare la composition solaire suivante de la même façon qu'à l'exemple A :

A Phase grasse

| | |
|---|---|
| - Alcool cétylstéarylique | 5,6 g |
| - Alcool cétylstéarylique polyoxyéthyléné à 33 moles d'oxyde d'éthylène | 1,4 g |
| - Mélange de monostéarate et de distéarate de glycéryle non autoémulsionnable | 2,0 g |
| - Huile de silicone | 1,5 g |
| - Alcool cétylique | 1,5 g |
| - Adipate de diisopropyle | 15,0 g |
| - 2,4,6-tris[p-(2'-éthylhexyl-1'-oxycarbonyl)anilino] 1,3,5-triazine vendue par la société BASF sous la dénomination "UVINUL T 150" | 1,5 g |
| - Glycérine | 20,0 g |
| - Conservateur, antioxydant | qs |
| TOTAL | 48,7 g |

B - Phase aqueuse

| | |
|---|---|
| - Acide benzène 1,4[di(3-méthylidène camphométhyl sulfonique)], sel d'aluminium | 5,1 g |
| - Triéthanolamine | 1,7 g |
| - Parfum, conservateur | qs |
| - Eau | 43,7 g |

## Revendications

**Revendications pour les Etats contractants suivants : AT, BE, CH, DE, DK, FR, GB, GR, IT, LI, NL, SE**

**1.** Emulsion cosmétique filtrant les radiations ultraviolettes de longueurs d'onde comprises entre 280 nm et 400 nm, caractérisée par le fait qu'elle comprend, dans la phase huileuse, la 2,4,6-tris[p-(2'-éthylhexyl-1'-oxycarbonyl)anilino]-1,3,5-triazine et, dans la phase aqueuse, l'acide benzène 1,4-[di(3-méthylidène camphométhyl sulfonique)], partiellement ou totalement neutralisé, de formule :

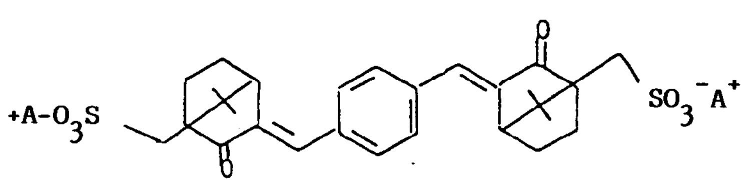

(I)

dans laquelle A désigne un atome d'hydrogène, un métal alcalin, un groupement $N(R)_4^+$, R désignant un atome d'hydrogène ou un radical alkyle ou hydroxyalkyle en $C_1$-$C_4$, ou encore un groupement $\frac{M^{n+}}{n}$ où $M^{n+}$

est un cation métallique polyvalent dans lequel n est égal à 2 ou 3 ou 4, $M^{n+}$ désignant de préférence $Ca^{2+}$, $Zn^{2+}$, $Mg^{2+}$, $Ba^{2+}$, $Al^{3+}$ ou $Zr^{4+}$.

**2.** Emulsion cosmétique filtrante selon la revendication 1, caractérisée par le fait qu'elle comprend 0,1 à 10% en poids, calculés sur la base de l'acide, d'acide benzène 1,4-[di(3-méthylidène camphométhyl sulfonique)], partiellement ou totalement neutralisé, et 0,1 à 10% en poids de 2,4,6-tris[p-(2'-éthylhexyl-1'-oxycarbonyl)anilino]-1,3,5-triazine.

**3.** Emulsion cosmétique filtrante selon la revendication 1 ou 2, caractérisée par le fait qu'elle comprend 1 à 6% en poids, calculés sur la base de l'acide, d'acide benzène 1,4-[di(3-méthylidène camphométhyl sulfonique)], partiellement ou totalement neutralisé et 1 à 6% en poids de 2,4,6-tris[p-(2'-éthylhexyl-1'-oxycarbonyl)anilino]-1,3,5-triazine.

**4.** Emulsion cosmétique filtrante selon l'une quelconque des revendications 1 à 3, caractérisée par le fait que l'acide benzène 1,4-[di(3-méthylidène camphométhyl sulfonique)] est partiellement ou totalement neutralisé par un hydroxyde alcalin, l'ammoniaque, une alcanolamine ou par un sel ou un hydroxyde de métal polyvalent de valence 2 ou 3 ou 4, choisi parmi le calcium, le zinc, le magnésium, le baryum, l'aluminium et le zirconium.

**5.** Emulsion cosmétique filtrante selon l'une quelconque des revendications 1 à 4, caractérisée par le fait qu'il s'agit d'une émulsion huile-dans-l'eau et que son pH est compris entre 4 et 9, et de préférence entre 5,5 et 8.

**6.** Emulsion cosmétique filtrante selon l'une quelconque des revendications 1 à 5, caractérisée par le fait que la phase huileuse est constituée de corps gras choisis parmi les huiles minérales, les huiles végétales ou animales modifiées ou non modifiées, les esters d'acides gras saturés ou insaturés, les huiles de synthèse, les cires, les huiles de silicone, les acides gras et alcools gras en $C_{12}$-$C_{18}$.

**7.** Composition cosmétique selon l'une quelconque des revendications 1 à 6, caractérisée par le fait que la phase aqueuse comprend outre de l'eau, des monoalcools ou polyalcools inférieurs en $C_1$-$C_6$.

**8.** Emulsion cosmétique filtrante selon l'une quelconque des revendications 1 à 7, caractérisée par le fait qu'elle contient en outre des émulsionnants à raison de 1 à 15% du poids total de l'émulsion.

**9.** Emulsion cosmétique filtrante selon l'une quelconque des revendications 1 à 8, caractérisée par le fait que les émulsionnants sont choisis parmi les alkyl ($C_8$-$C_{12}$) phénols polyoxyéthylénés contenant 9 à 15 moles d'oxyde d'éthylène; les alcools gras en $C_{12}$-$C_{18}$ polyoxyéthylénés comportant au moins 4 et de préférence 4 à 35 moles d'oxyde d'éthylène; les alcools gras en $C_{10}$-$C_{18}$ polyglycérolés comportant 4 à 10 motifs glycérol; les alcools gras en $C_{12}$-$C_{18}$ polyoxypropylénés comportant de préférence 3 moles d'oxyde de propylène; les esters d'acides gras en $C_{12}$-$C_{18}$ à la fois polyoxyéthylénés et polyglycérolés ou monoglycérolés; les esters d'acides gras en $C_{12}$-$C_{18}$ du sorbitan, polyoxyéthylénés, contenant 10 à 20 moles d'oxyde d'éthylène; les esters d'acides gras en $C_{12}$-$C_{18}$ polyoxyéthylénés comportant au moins 2 motifs d'oxyde d'éthylène et de préférence 4 à 20 motifs d'oxyde d'éthylène; l'huile de ricin polyoxyéthylénée contenant 10 à 60 moles d'oxyde d'éthylène; les copolymères oxyde de propylène/oxyde d'éthylène; la lécithine de soja ou de jaune d'oeuf; les sucroglycérides; les savons; les esters phosphoriques d'alcools gras; les sulfates d'alcools gras éventuellement oxyéthylénés; les alcools de lanoline polyoxyéthylénés et comportant au moins 4 motifs d'oxyde d'éthylène.

**10.** Composition cosmétique filtrante selon l'une quelconque des revendications 1 à 9, caractérisée par le fait qu'elle contient en outre des épaississants, des adoucissants, des humectants, des tensio-actifs, des conservateurs, des anti-mousses, des parfums, des colorants et des pigments.

**11.** Emulsion cosmétique filtrante selon l'une quelconque des revendications 1 à 10, caractérisée par le fait qu'elle se présente sous forme de lait, crème ou est conditionnée en aérosol.

**12.** Emulsion cosmétique filtrante selon l'une quelconque des revendications 1 à 11, caractérisée par le fait qu'elle comprend en outre d'autres filtres UV hydrosolubles ou liposolubles choisis parmi l'huile de café, les dérivés de l'acide salicylique, les dérivés de l'acide cinnamique, les esters et dérivés de l'acide p-amino benzoïque, les anthranilates, les dérivés de benzophénone, les dérivés du benzylidène camphre tels que

le p-méthylbenzylidène camphre, les dérivés du dibenzoylméthane, les dérivés du benzotriazole, les dérivés du benzoxazole, les dérivés du benzimidazole et les α-cyano β,β-diphényl acrylates d'alkyle ($C_2$-$C_{10}$).

13. Procédé de traitement cosmétique de l'épiderme humain en vue de sa protection contre les radiations ultraviolettes de longueurs d'ondes comprises entre 280 et 400 nm, caractérisé par le fait qu'il consiste à appliquer sur la peau une quantité efficace d'une émulsion cosmétique filtrante selon l'une quelconque des revendications 1 à 12.

**Revendications pour l'Etat contractant suivant : ES**

1. Emulsion cosmétique filtrant les radiations ultraviolettes de longueurs d'onde comprises entre 280 nm et 400 nm, caractérisée par le fait qu'elle comprend, dans la phase huileuse, la 2,4,6-tris[p- (2'-éthylhexyl-1'-oxycarbonyl)anilino]-1,3,5-triazine et, dans la phase aqueuse, l'acide benzène 1,4-[di(3-méthylidène camphométhyl sulfonique)], partiellement ou totalement neutralisé, de formule :

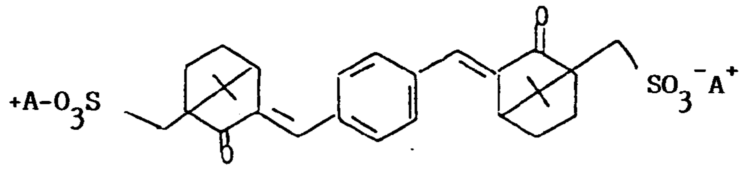

(I)

dans laquelle A désigne un atome d'hydrogène, un métal alcalin, un groupement $N(R)_4^+$, R désignant un atome d'hydrogène ou un radical alkyle ou hydroxyalkyle en $C_1$-$C_4$, ou encore un groupement $\frac{M^{n+}}{n}$ où $M^{n+}$ est un cation métallique polyvalent dans lequel n est égal à 2 ou 3 ou 4, $M^{n+}$ désignant de préférence $Ca^{2+}$, $Zn^{2+}$, $Mg^2$, $Ba^{2+}$, $Al^{3+}$ ou $Zr^{4+}$.

2. Emulsion cosmétique filtrante selon la revendication 1, caractérisée par le fait qu'elle comprend 0,1 à 10% en poids, calculés sur la base de l'acide, d'acide benzène 1,4-[di(3-méthylidène camphométhyl sulfonique)], partiellement ou totalement neutralisé, et 0,1 à 10% en poids de 2,4,6-tris[p-(2'-éthylhexyl-1'-oxycarbonyl)anilino]-1,3,5-triazine.

3. Emulsion cosmétique filtrante selon la revendication 1 ou 2, caractérisée par le fait qu'elle comprend 1 à 6% en poids, calculés sur la base de l'acide, d'acide benzène 1,4-[di(3-méthylidène camphométhyl sulfonique)], partiellement ou totalement neutralisé et 1 à 6% en poids de 2,4,6-tris[p-(2'-éthylhexyl-1'-oxycarbonyl)anilino]-1,3, 5-triazine.

4. Emulsion cosmétique filtrante selon l'une quelconque des revendications 1 à 3, caractérisée par le fait que l'acide benzène 1,4-[di(3-méthylidène camphométhyl sulfonique)] est partiellement ou totalement neutralisé par un hydroxyde alcalin, l'ammoniaque, une alcanolamine ou par un sel ou un hydroxyde de métal polyvalent de valence 2 ou 3 ou 4, choisi parmi le calcium, le zinc, le magnésium, le baryum, l'aluminiumet le zirconium.

5. Emulsion cosmétique filtrante selon l'une quelconque des revendications 1 à 4, caracterisée par le fait qu'il s'agit d'une émulsion huile-dans-l'eau et que son pH est compris entre 4 et 9, et de préférence entre 5,5 et 8.

6. Emulsion cosmétique filtrante selon l'une quelconque des revendications 1 à 5, caractérisée par le fait que la phase huileuse est constituée de corps gras choisis parmi les huiles minérales, les huiles végétales ou animales modifiées ou non modifiées, les esters d'acides gras saturés ou insaturés, les huiles de synthèse, les cires, les huiles de silicone, les acides gras et alcools gras en $C_{12}$-$C_{18}$.

7. Composition cosmétique selon l'une quelconque des revendications 1 à 6, caractérisée par le fait que la phase aqueuse comprend outre de l'eau, des monoalcools ou polyalcools inférieurs en $C_1$-$C_6$.

8. Emulsion cosmétique filtrante selon l'une quelconque des revendications 1 à 7, caractérisée par le fait

qu'elle contient en outre des émulsionnants à raison de 1 à 15% du poids total de l'émulsion.

9. Emulsion cosmétique filtrante selon l'une quelconque des revendications 1 à 8, caractérisée par le fait que les émulsionnants sont choisis parmi les alkyl ($C_8$-$C_{12}$) phénols polyoxyéthylénés contenant 9 à 15 moles d'oxyde d'éthylène; les alcools gras en $C_{12}$-$C_{18}$ polyoxyéthylénés comportant au moins 4 et de préférence 4 à 35 moles d'oxyde d'éthylène; les alcools gras en $C_{10}$-$C_{18}$ polyglycérolés comportant 4 à 10 motifs glycérol; les alcools gras en $C_{12}$-$C_{18}$ polyoxypropylénés comportant de préférence 3 moles d'oxyde de propylène; les esters d'acides gras en $C_{12}$-$C_{18}$ à la fois polyoxyéthylénés et polyglycérolés ou monoglycérolés; les esters d'acides gras en $C_{12}$-$C_{18}$ du sorbitan, polyoxyéthylénés, contenant 10 à 20 moles d'oxyde d'éthylène; les esters d'acides gras en $C_{12}$-$C_{18}$ polyoxyéthylénés comportant au moins 2 motifs d'oxyde d'éthylène et de préférence 4 à 20 motifs d'oxyde d'éthylène; l'huile de ricin polyoxyéthylénée contenant 10 à 60 moles d'oxyde d'éthylène; les copolymères oxyde de propylène/oxyde d'éthylène; la lécithine de soja ou de jaune d'oeuf; les sucroglycérides; les savons; les esters phosphoriques d'alcools gras; les sulfates d'alcools gras éventuellement oxyéthylénés; les alcools de lanoline polyoxyéthylénés et comportant au moins 4 motifs d'oxyde d'éthylène.

10. Composition cosmétique filtrante selon l'une quelconque des revendications 1 à 9, caractérisée par le fait qu'elle contient en outre des épaississants, des adoucissants, des humectants, des tensio-actifs, des conservateurs, des anti-mousses, des parfums, des colorants et des pigments.

11. Emulsion cosmétique filtrante selon l'une quelconque des revendications 1 à 10, caractérisée par le fait qu'elle se présente sous forme de lait, crème ou est conditionnée en aérosol.

12. Emulsion cosmétique filtrante selon l'une quelconque des revendications 1 à 11, caractérisée par le fait qu'elle comprend en outre d'autres filtres UV hydrosolubles ou liposolubles choisis parmi l'huile de café, les dérivés de l'acide salicylique, les dérivés de l'acide cinnamique, les esters et dérivés de l'acide p-amino benzoïque, les anthranilates, les dérivés de benzophénone, les dérivés du benzylidène camphre tels que le p-méthylbenzylidène camphre, les dérivés du dibenzoylméthane, les dérivés du benzotriazole, les dérivés du benzoxazole, les dérivés du benzimidazole et les $\alpha$-cyano $\beta$,$\beta$-diphényl acrylates d'alkyle ($C_2$-$C_{10}$).

13. Procédé de traitement cosmétique de l'épiderme humain en vue de sa protection contre les radiations ultraviolettes de longueurs d'ondes comprises entre 280 et 400 nm, caractérisé par le fait qu'il consiste à appliquer sur la peau une quantité efficace d'une émulsion cosmétique filtrante selon l'une quelconque des revendications 1 à 12.

14. Procédé de préparation d'une émulsion cosmétique filtrant les rayons UV de longueurs d'onde comprises entre 280 et 400 nm, caractérisé en ce qu'il consiste à incorporer dans la phase aqueuse de l'émulsion pouvant contenir des monoalcools ou polyalcools inférieurs contenant 1 à 6 atomes de carbone, 0,1 à 10% en poids par rapport au poids total de la composition, d'acide benzène 1,4-[di(3-méthylidène camphométhyl sulfonique)], de formule (I) selon la revendication 1, partiellement ou totalement neutralisé, à incorporer dans la phase huileuse de l'émulsion, constituée de corps gras choisis parmi les huiles minérales, les huiles végétales ou animales, modifiées ou non modifiées, les esters d'acide gras saturés ou insaturés, les huiles de synthèse, les cires, les huiles de silicone, les acides gras et alcools gras en $C_{12}$-$C_{18}$, 0,1 à 10% en poids de 2,4,6-tris[p-(2'-éthylhexyl-1'-oxycarbonyl) anilino]-1,3,5- triazine, et à ajouter des émulsionnants et éventuellement au moins un adjuvant cosmétique choisi parmi les épaississants, les adoucissants, les humectants, les tensio-actifs, les conservateurs, les anti-mousses, les parfums, les colorants, les pigments et d'autres filtres UV.

## Patentansprüche

### Patentansprüche für folgende Vertragsstaaten : AT, BE, CH, DE, FR, GB, GR, IT, LI, SE, DK, NL

1. Kosmetische Emulsion, die UV-Strahlen einer Wellenlänge von 280 bis 400 nm filtert, dadurch **gekennzeichnet**, daß sie, in der Ölphase, 2,4,6-Tris(p-(2'-ethylhexyl-1'-oxycarbonyl)anilino)-1,3,5-triazin und, in der wässrigen Phase, 1,4-Di(3-methylidenkamphomethylsulfonsäure)benzol, teilweise oder ganz neutralisiert, der Formel enthält:

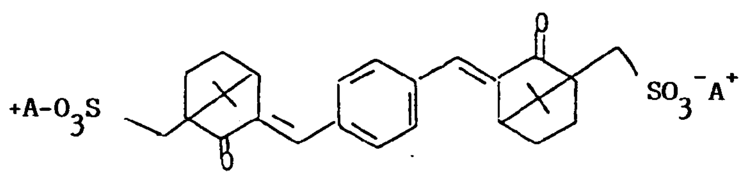 (I)

worin A ein Wasserstoffatom, ein Alkalimetall, eine Gruppe $N(R)_4^+$ , wobei R ein Wasserstoffatom oder einen $C_{1-4}$-Alkyl- oder -Hydroxyalkylrest bezeichnet, oder auch eine Gruppe $M^{n+}/n$ darstellt, worin $M^{n+}$ ein mehrwertiges Metallkation mit n gleich 2 oder 3 oder 4 ist, wobei $M^{n+}$ vorzugsweise $Ca^{2+}$, $Zn^{2+}$, $Mg^{2+}$, $Ba^{2+}$, $Al^{3+}$ oder $Zr^{4+}$ bedeutet.

**2.** Kosmetische Filteremulsion gemäß Anspruch 1,
dadurch **gekennzeichnet**, daß
sie 0,1 bis 10 Gew.%, berechnet auf Basis der Säure, 1,4-Di(3-methylidenkamphomethylsulfonsäure)benzol, teilweise oder ganz neutralisiert, und 0,1 bis 10 Gew.% 2,4,6-Tris(p-(2'-ethylhexyl-1'-oxycarbonyl)anilino)-1,3,5-triazin enthält.

**3.** Kosmetische Filteremulsion gemäß Anspruch 1 oder 2,
dadurch **gekennzeichnet**, daß
sie 1 bis 6 Gew.%, berechnet auf Basis der Säure, 1,4(Di(3-methylidenkamphomethylsulfonsäure)benzol, teilweise oder ganz neutralisiert, und 1 bis 6 Gew. % 2,4,6-Tris(p-(2'-ethylhexyl-1'-oxycarbonyl)anilino)-1,3- 5-triazin enthält.

**4.** Kosmetische Filteremulsion gemäß jedem Anspruch 1 bis 3,
dadurch **gekennzeichnet**, daß
das 1,4-Di(3-methylidenkamphomethylsulfonsäure)benzol teilweise oder ganz durch ein Alkalihydroxid, Ammoniak, ein Alkanolamin oder durch ein Salz oder Hydroxid eines mehrwertigen Metalls der Wertigkeit 2 oder 3 oder 4, ausgewählt aus Calcium, Zink, Magnesium, Barium, Aluminium und Zirkon, neutralisiert ist.

**5.** Kosmetische Filteremulsion gemäß jedem Anspruch 1 bis 4,
dadurch **gekennzeichnet**, daß
es sich um eine Öl-in-Wasser-Emulsion handelt und ihr pH 4 bis 9, vorzugsweise 5,5 bis 8, beträgt.

**6.** Kosmetische Filteremulsion gemäß jedem Anspruch 1 bis 5,
dadurch **gekennzeichnet**, daß
die Ölphase aus Fettkörpern zusammengesetzt ist, ausgewählt aus Mineralölen, modifizierten oder nicht modifizierten pflanzlichen oder tierischen Ölen, gesättigten oder ungesättigten Fettsäureestern, Syntheseölen, Wachsen, Siliconölen, $C_{12-18}$-Fettsäuren und -Fettalkoholen.

**7.** Kosmetische Zusammensetzung gemäß jedem Anspruch 1 bis 6,
dadurch **gekennzeichnet**, daß
die wässrige Phase ausser Wasser $C_{1-6}$-Niedrigmonoalkohole oder -polyalkohole enthält.

**8.** Kosmetische Filteremulsion gemäß jedem Anspruch 1 bis 7,
dadurch **gekennzeichnet**, daß
sie ausserdem Emulgatoren mit 1 bis 15% des Gesamtgewichts der Emulsion enthält.

**9.** Kosmetische Filteremulsion gemäß jedem Anspruch 1 bis 8,
dadurch **gekennzeichnet**, daß
die Emulgatoren ausgewählt sind aus polyoxethylierten $C_{8-12}$-Alkylphenolen, enthaltend 9 bis 15 Mol Ethylenoxid; polyoxyethylierten $C_{12-18}$-Fettalkoholen, enthaltend mindestens 4 und vorzugsweise 4 bis 35 Mol Ethylenoxid; polyglycerierten $C_{10-18}$-Fettalkoholen, enthaltend 4 bis 10 Glycerin-Einheiten; polyoxpropylierten $C_{12-18}$-Fettalkoholen, enthaltend vorzugsweise 3 Mol Propylenoxid; $C_{12-18}$-Fettsäureestern, gleichzeitig polyoxethyliert und polyglyceriert oder monoglyceriert; Sorbitan-$C_{12-18}$-fettsäureestern, polyoxethyliert, enthaltend 10 bis 20 Mol Ethylenoxid; polyoxethylierten $C_{12-18}$-Fettsäu-

reestern, enthaltend mindestens 2 und vorzugsweise 4 bis 20 Ethylenoxid-Einheiten; polyoxyethyliertem Rizinusöl, enthaltend 10 bis 60 Mol Ethylenoxid; Copolymeren von Propylen-/Ethylenoxid; Lecithin von Soja oder Eigelb; Zuckerglyceriden, Seifen, Phosphorsäureestern von Fettalkoholen; gegebenenfalls oxethylierten Sulfaten von Fettalkoholen; Lanolinalkoholen, polyoxethyliert und enthaltend mindestens 4 Ethylenoxideinheiten.

**10.** Kosmetische Filterzusammensetzung gemäß jedem Anspruch 1 bis 9,
dadurch **gekennzeichnet**, daß
sie ausserdem Verdickungsmittel, Weichmacher, Feuchtigkeitsmittel, oberflächenaktive Mittel, Konservierungsmittel, Antischaummittel, Parfüms, Färbemittel und Pigmente enthält.

**11.** Kosmetische Filteremulsion gemäß jedem Anspruch 1 bis 10,
dadurch **gekennzeichnet**, daß
sie in Form einer Milch oder Creme vorliegt oder als Aerosol zubereitet ist.

**12.** Kometische Filteremulsion gemäß jedem Anspruch 1 bis 11,
dadurch **gekennzeichnet**, daß
sie ausserdem weitere wasser- oder fettlösliche UV-Filterstoffe enthält, ausgewählt aus Kaffeeöl, Salicylsäurederivaten, Zimtsäurederivaten, Estern und Derivaten von p-Aminobenzoesäure, Anthranilaten, Benzophenonderivaten, Benzylidenkampherderivaten wie p-Methylbenzylidenkampher, Dibenzoylmethanderivaten, Benzotriazolderivaten, Benzoxazolderivaten, Benzimidazolderivaten und $C_{2-10}$-Alkyl-$\alpha$-cyano-$\beta$,$\beta$-diphenylacrylaten.

**13.** Kosmetisches Behandlungsverfahren der menschlichen Haut zum Schutz gegen ultraviolette Bestrahlungen von Wellenlängen von 280 bis 400 nm,
dadurch **gekennzeichnet**, daß
man auf die Haut eine wirksame Menge einer kosmetischen Filteremulsion gemäß jedem Anspruch 1 bis 12 aufträgt.

**Patentansprüche für folgenden Vertragsstaat : ES**

**1.** Kosmetische Emulsion, die UV-Strahlen einer Wellenlänge von 280 bis 400 nm filtert,
dadurch **gekennzeichnet**, daß
sie, in der Ölphase, 2,4,6-Tris(p-(2'-ethylhexyl-1'-oxycarbonyl)anilino)-1,3,5-triazin und, in der wässrigen Phase, 1,4-Di(3-methylidenkamphomethylsulfonsäure)benzol, teilweise oder ganz neutralisiert, der Formel enthält:

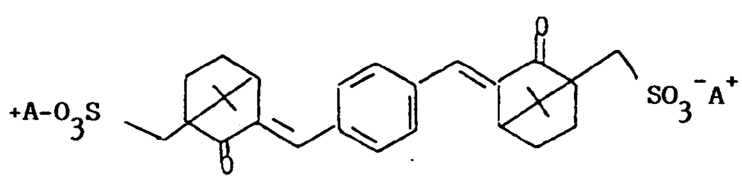
(I)

worin A ein Wasserstoffatom, ein Alkalimetall, eine Gruppe $N(R)_4^+$ , wobei R ein Wasserstoffatom oder einen $C_{1-4}$-Alkyl- oder -Hydroxyalkylrest bezeichnet, oder auch eine Gruppe $M^{n+}/n$ darstellt, worin $M^{n+}$ ein mehrwertiges Metallkation mit n gleich 2 oder 3 oder 4 ist, wobei $M^{n+}$ vorzugsweise $Ca^{2+}$, $Zn^{2+}$, $Mg^{2+}$, $Ba^{2+}$, $Al^{3+}$ oder $Zr^{4+}$ bedeutet.

**2.** Kosmetische Filteremulsion gemäß Anspruch 1,
dadurch **gekennzeichnet**, daß
sie 0,1 bis 10 Gew.%, berechnet auf Basis der Säure, 1,4-Di(3-methylidenkamphomethylsulfonsäure)benzol, teilweise oder ganz neutralisiert, und 0,1 bis 10 Gew.% 2,4,6-Tris(p-(2'-ethylhexyl-1'-oxycarbonyl)anilino)-1,3,5-triazin enthält.

**3.** Kosmetische Filteremulsion gemäß Anspruch 1 oder 2,
dadurch **gekennzeichnet**, daß

sie 1 bis 6 Gew.%, berechnet auf Basis der Säure, 1,4-Di(3-methylidenkamphomethylsulfonsäure)benzol, teilweise oder ganz neutralisiert, und 1 bis 6 Gew.%, 2,4,6-Tris(p-(2'-ethylhexyl-1'-oxycarbonyl)anilino)-1,3-5-triazin enthält.

**4.** Kosmetische Filteremulsion gemäß jedem Anspruch 1 bis 3,
dadurch **gekennzeichnet**, daß
das 1,4-Di(3-methylidenkamphomethylsulfonsäure)benzol teilweise oder ganz durch ein Alkalihydroxid, Ammoniak, ein Alkanolamin oder durch ein Salz oder Hydroxid eines mehrwertigen Metalls der Wertigkeit 2 oder 3 oder 4, ausgewählt aus Calcium, Zink, Magnesium, Barium, Aluminium und Zirkon, neutralisiert ist.

**5.** Kosmetische Filteremulsion gemäß jedem Anspruch 1 bis 4,
dadurch **gekennzeichnet**, daß
es sich um eine Öl-in-Wasser-Emulsion handelt und ihr pH 4 bis 9, vorzugsweise 5,5 bis 8, beträgt.

**6.** Kosmetische Filteremulsion gemäß jedem Anspruch 1 bis 5,
dadurch **gekennzeichnet**, daß
die Ölphase aus Fettkörpern zusammengesetzt ist, ausgewählt aus Mineralölen, modifizierten oder nicht modifizierten pflanzlichen oder tierischen Ölen, gesättigten oder ungesättigten Fettsäureestern, Syntheseölen, Wachsen, Siliconölen, $C_{12-18}$-Fettsäuren und -Fettalkoholen.

**7.** Kosmetische Zusammensetzung gemäß jedem Anspruch 1 bis 6,
dadurch **gekennzeichnet**, daß
die wässrige Phase ausser Wasser $C_{1-6}$-Niedrigmonoalkohole oder -polyalkohole enthält.

**8.** Kosmetische Filteremulsion gemäß jedem Anspruch 1 bis 7,
dadurch **gekennzeichnet**, daß
sie ausserdem Emulgatoren mit 1 bis 15% des Gesamtgewichts der Emulsion enthält.

**9.** Kosmetische Filteremulsion gemäß jedem Anspruch 1 bis 8,
dadurch **gekennzeichnet**, daß
die Emulgatoren ausgewählt sind aus polyoxethylierten $C_{8-12}$-Alkylphenolen, enthaltendn 9 bis 15 Mol Ethylenoxid; polyoxyethylierten $C_{12-18}$-Fettalkoholen, enthaltend mindestens 4 und vorzugsweise 4 bis 35 Mol Ethylenoxid; polyglycerierten $C_{10-18}$-Fettalkoholen, enthaltend 4 bis 10 Glycerin-Einheiten; polyoxpropylierten $C_{12-18}$-Fettalkoholen, enthaltend vorzugsweise 3 Mol Propylenoxid; $C_{12-18}$-Fettsäureestern, gleichzeitig polyoxethyliert und polyglyceriert oder monoglyceriert; Sorbitan-$C_{12-18}$-fettsäureestern, polyoxethyliert, enthaltend 10 bis 20 Mol Ethylenoxid; polyoxethylierten $C_{12-18}$-Fettsäureestern, enthaltend mindestens 2 und vorzugsweise 4 bis 20 Ethylenoxid-Einheiten; polyoxyethyliertem Rizinusöl, enthaltend 10 bis 60 Mol Ethylenoxid; Copolymeren von Propylen/Ethylenoxid; Lecithin von Soja oder Eigelb; Zuckerglyceriden, Seifen, Phosphorsäureestern von Fettalkoholen; gegebenenfalls oxethylierten Sulfaten von Fettalkoholen; Lanolinalkoholen, polyoxethyliert und enthaltend mindestens 4 Ethylenoxideinheiten.

**10.** Kosmetische Filterzusammensetzung gemäß jedem Anspruch 1 bis 9,
dadurch **gekennzeichnet**, daß
sie ausserdem Verdickungsmittel, Weichmacher, Feuchtigkeitsmittel, oberflächenaktive Mittel, Konservierungsmittel, Antischaummittel, Parfüms, Färbemittel und Pigmente enthält.

**11.** Kosmetische Filteremulsion gemäß jedem Anspruch 1 bis 10,
dadurch **gekennzeichnet**, daß
sie in Form einer Milch oder Creme vorliegt oder als Aerosol zubereitet ist.

**12.** Kometische Filteremulsion gemäß jedem Anspruch 1 bis 11,
dadurch **gekennzeichnet**, daß
sie ausserdem weitere wasser- oder fettlösliche UV-Filterstoffe enthält, ausgewählt aus Kaffeeöl, Salicylsäurederivaten, Zimtsäurederivaten, Estern und Derivaten von p-Aminobenzoesäure, Anthranilaten, Benzophenonderivaten, Benzylidenkampherderivaten wie p-Methylbenzylidenkampher, Dibenzoylmethanderivaten, Benzotriazolderivaten, Benzoxazolderivaten, Benzimidazolderivaten und $C_{2-10}$-Alkyl-$\alpha$-cyano-$\beta$,$\beta$-diphe-

nylacrylaten.

13. Kosmetisches Behandlungsverfahren der menschlichen Haut zum Schutz gegen ultraviolette Bestrahlungen von Wellenlängen von 280 bis 400 nm,
dadurch **gekennzeichnet**, daß
man auf die Haut eine wirksame Menge einer kosmetischen Filteremulsion gemäß jedem Anspruch 1 bis 12 aufträgt.

14. Verfahren zur Herstellung einer kosmetischen Emulsion, die die UV-Strahlen von Wellenlängen von 280 bis 400 nm filtert,
dadurch **gekennzeichnet**, daß
man in die wässrige Phase der Emulsion, die Niedrigmonoalkohole oder -polyalkohole mit 1 bis 6 Kohlenstoffatomen enthalten kann, 0,1 bis 10 Gew.%, bezogen auf Gesamtgewicht der Zusammensetzung, 1,4-Di(3-methylidenkamphomethylsulfonsäure) benzol der Formel (I) gemäß Anspruch 1,teilweise oder ganz neutralisiert, in die Ölphase der Emulsion, die zusammengesetzt ist aus Fettkörpern, ausgewählt aus Mineralölen, pflanzlichen oder tierischen Ölen, modifiziert oder nicht modifiziert, gesättigten oder ungesättigten Fettsäureestern, Syntheseölen, Wachsen, Siliconölen, $C_{12-18}$-Fettsäuren und -Fettalkoholen, 0,1 bis 10 Gew.% 2,4,6-Tris (p-(2'-ethylhexyl-1'-oxycarbonyl)anilino)-1,3,5-triazin einbringt und Emulgatoren und gegebenenfalls mindestens einen kosmetischen Hilfstoff zufügt, ausgewählt aus Verdickungsmitteln, Weichmachern, Feuchtigkeitsmitteln, oberflächenaktiven Mitteln, Konservierungsmitteln, Antischaummitteln, Parfüms, Färbemitteln, Pigmenten und weiteren UV-Filterstoffen.

## Claims

**Claims for the following Contracting States : AT, BE, CH, DE, DK, FR, GB, GR, IT, LI, SE, NL**

1. Cosmetic emulsion screening out ultraviolet radiation of wavelengths between 280 nm and 400 nm characterised in that it comprises in the oil phase, 2,4,6-tris[p-(2'-ethylhexyl-1'-oxycarbonyl)anilino]-1,3,5-triazine and, in the aqueous phase, benzene-1,4-[bis(3-methylidenecamphormethylsulphonic)] acid, partially or completely neutralised, of formula:

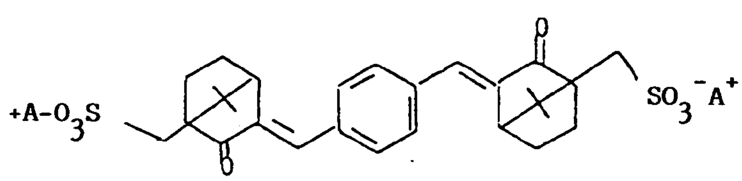
(I)

in which A designates a hydrogen atom, an alkali metal, a $N(R)_4^+$ group, R designating a hydrogen atom or a $C_1$-$C_4$ alkyl or hydroxyalkyl radical, or a $\frac{M^{n+}}{n}$ group where $M^{n+}$ is a polyvalent metal cation in which n is equal to 2 or 3 or 4, $M^{n+}$ preferably designating $Ca^{2+}$, $Zn^{2+}$, $Mg^{2+}$, $Ba^{2+}$, $Al^{3+}$ or $Zr^{4+}$.

2. Cosmetic screening emulsion according to Claim 1, characterised in that it comprises 0.1 to 10% by weight, calculated on the basis of the acid, benzene-1,4-[bis-(3-methylidenecamphormethylsulphonic)] acid, partially or completely neutralised, and 0.1 to 10% by weight of 2,4,6-tris[p-(2'-ethylhexyl-1'-oxycarbonyl)anilino]-1,3,5-triazine.

3. Cosmetic screening emulsion according to Claim 1 or 2, characterised in that it comprises 1 to 6% by weight, calculated on the basis of the acid, benzene-1,4-[bis(3-methylidenecamphormethylsulphonic)] acid, partially or completely neutralised, and 1 to 6% by weight of 2,4,6-tris[p-(2'-ethylhexyl-1'-oxycarbonyl)-anilino]-1,3,5-triazine.

4. Cosmetic screening emulsion according to any one of Claims 1 to 3, characterised in that the benzene-1,4-[bis(3-methylidenecamphormethylsulphonic)] acid is partially or completely neutralised by an alkali hydroxide, ammonium hydroxide, an alkanolamine or by a polyvalent metal salt or hydroxide of valency

equal to 2 or 3 or 4, chosen from among calcium, zinc, magnesium, barium, aluminium and zirconium.

5. Cosmetic screening emulsion according to any one of Claims 1 to 4, characterised in that it applies to an oil-in-water emulsion and that its pH is between 4 and 9, and preferably between 5.5 and 8.

6. Cosmetic screening emulsion according to any one of Claims 1 to 5, characterised in that the oil phase consists of fatty substances chosen from among mineral oils, modified or non-modified vegetable or animal oils, saturated or unsaturated fatty acid esters, synthetic oils, waxes, silicone oils and $C_{12}$-$C_{18}$ fatty acids and fatty alcohols.

7. Cosmetic composition according to any one of Claims 1 to 6, characterised in that the aqueous phase comprises in addition to water $C_1$-$C_6$ lower monoalcohols or polyalcohols.

8. Cosmetic screening emulsion according to any one of Claims 1 to 7, characterised in that it contains in addition emulsifiers in an amount of 1 to 15% of the total weight of the emulsion.

9. Cosmetic screening emulsion according to any one of Claims 1 to 8, characterised in that the emulsifiers are chosen from among polyoxyethylenated alkyl ($C_8$-$C_{12}$) phenols containing 9 to 15 moles of ethylene oxide; polyoxyethylenated $C_{12}$-$C_{18}$ fatty alcohols comprising at least 4 and preferably 4 to 35 moles of ethylene oxide; polyglycerolated $C_{10}$-$C_{18}$ fatty alcohols comprising 4 to 10 glycerol residues; polyoxypropylenated $C_{12}$-$C_{18}$ fatty alcohols preferably comprising 3 moles of propylene oxide; $C_{12}$-$C_{18}$ fatty acid esters conjointly polyoxyethylenated and polyglycerolated or monoglycerolated; polyoxyethylenated $C_{12}$-$C_{18}$ fatty acid esters of sorbitan containing 10 to 20 moles of ethylene oxide; polyoxyethylenated $C_{12}$-$C_{18}$ fatty acid esters comprising at least 2 ethylene oxide residues and preferably 4 to 20 ethylene oxide residues; polyoxyethylenated castor oil containing 10 to 60 moles of ethylene oxide; propylene oxide/ethylene oxide copolymers; soybean or egg yolk lecithin; sucroglycerides; soaps; fatty alcohol phosphoric esters; fatty alcohol sulphates optionally oxyethylenated; lanolin alcohols, polyoxyethylenated and comprising at least 4 ethylene oxide residues.

10. Cosmetic screening composition according to any one of Claims 1 to 9, characterised in that it contains in addition thickeners, emollients, humectants, surface-active agents, preservatives, antifoams, perfumes, dyes and pigments.

11. Cosmetic screening emulsion according to any one of Claims 1 to 10, characterised in that it is provided in the form of a milk or cream or is prepared as an aerosol.

12. Cosmetic screening emulsion according to any one of Claims 1 to 11, characterised in that it comprises in addition other water soluble or fat soluble UV screens chosen from among coffee oil, salicylic acid derivatives, cinnamic acid derivatives, p-aminobenzoic acid esters and derivatives, anthranilates, benzophenone derivatives, benzylidenecamphor derivatives such as p-methylbenzylidenecamphor, dibenzoylmethane derivatives, benzotriazole derivatives, benzoxazole derivatives, benzimidazole derivatives and alkyl($C_2$-$C_{10}$) $\alpha$-cyano-$\beta$,$\beta$-diphenylacrylates.

13. Process for cosmetic treatment of the human epidermis with a view to protecting it against ultraviolet radiation of wavelengths between 280 and 400 nm, characterised in that it consists in applying to the skin an efficacious quantity of a cosmetic screening emulsion according to any one of Claims 1 to 12.

**Claims for the following Contracting State : ES**

1. Cosmetic emulsion screening out ultraviolet radiation of wavelengths between 280 nm and 400 nm characterised in that it comprises in the oil phase, 2,4,6-tris[p-(2'-ethylhexyl-1'-oxycarbonyl)anilino]-1,3,5-triazine and, in the aqueous phase, benzene-1,4-[bis(3-methylidenecamphormethylsulphonic)] acid, partially or completely neutralised, of formula:

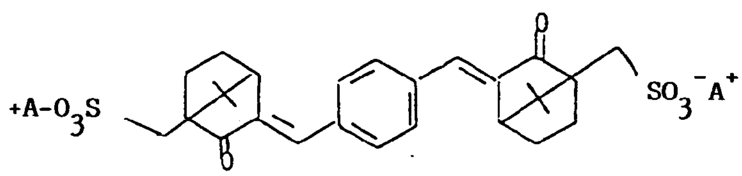 (I)

in which A designates a hydrogen atom, an alkali metal, a $N(R)_4^+$ group, R designating a hydrogen atom or a $C_1$-$C_4$ alkyl or hydroxyalkyl radical, or a $\frac{M^{n+}}{n}$ group where $M^{n+}$ is a polyvalent metal cation in which n is equal to 2 or 3 or 4, $M^{n+}$ preferably designating $Ca^{2+}$, $Zn^{2+}$, $Mg^{2+}$, $Ba^{2+}$, $Al^{3+}$ or $Zr^{4+}$.

2. Cosmetic screening emulsion according to Claim 1, characterised in that it comprises 0.1 to 10% by weight, calculated on the basis of the acid, benzene-1,4-[bis-(3-methylidenecamphormethylsulphonic)] acid, partially or completely neutralised, and 0.1 to 10% by weight of 2,4,6-tris[p-(2'-ethylhexyl-1'-oxycarbonyl)anilino]-1,3,5-triazine.

3. Cosmetic screening emulsion according to Claim 1 or 2, characterised in that it comprises 1 to 6% by weight, calculated on the basis of the acid, benzene-1,4-[bis (3-methylidenecamphormethylsulphonic)] acid, partially or completely neutralised, and 1 to 6% by weight of 2,4,6-tris[p-(2'-ethylhexyl-1'-oxycarbonyl)-anilino]-1,3,5-triazine.

4. Cosmetic screening emulsion according to any one of Claims 1 to 3, characterised in that the benzene-1,4-[bis(3-methylidenecamphormethylsulphonic)] acid is partially or completely neutralised by an alkali hydroxide, ammonium hydroxide, an alkanolamine or by a polyvalent metal salt or hydroxide of valency equal to 2 or 3 or 4, chosen from among calcium, zinc, magnesium, barium, aluminium and zirconium.

5. Cosmetic screening emulsion according to any one of Claims 1 to 4, characterised in that it applies to an oil-in-water emulsion and that its pH is between 4 and 9, and preferably between 5.5 and 8.

6. Cosmetic screening emulsion according to any one of Claims 1 to 5, characterised in that the oil phase consists of fatty substances chosen from among mineral oils, modified or non-modified vegetable or animal oils, saturated or unsaturated fatty acid esters, synthetic oils, waxes, silicone oils and $C_{12}$-$C_{18}$ fatty acids and fatty alcohols.

7. Cosmetic composition according to any one of Claims 1 to 6, characterised in that the aqueous phase comprises in addition to water $C_1$-$C_6$ lower monoalcohols or polyalcohols.

8. Cosmetic screening emulsion according to any one of Claims 1 to 7, characterised in that it contains in addition emulsifiers in an amount of 1 to 15% of the total weight of the emulsion.

9. Cosmetic screening emulsion according to any one of Claims 1 to 8, characterised in that the emulsifiers are chosen from among polyoxyethylenated alkyl ($C_8$-$C_{12}$) phenols containing 9 to 15 moles of ethylene oxide; polyoxyethylenated $C_{12}$-$C_{18}$ fatty alcohols comprising at least 4 and preferably 4 to 35 moles of ethylene oxide; polyglycerolated $C_{10}$-$C_{18}$ fatty alcohols comprising 4 to 10 glycerol residues; polyoxypropylenated $C_{12}$-$C_{18}$ fatty alcohols preferably comprising 3 moles of propylene oxide; $C_{12}$-$C_{18}$ fatty acid esters conjointly polyoxyethylenated and polyglycerolated or monoglycerolated; polyoxyethylenated $C_{12}$-$C_{18}$ fatty acid esters of sorbitan containing 10 to 20 moles of ethylene oxide; polyoxyethylenated $C_{12}$-$C_{18}$ fatty acid esters comprising at least 2 ethylene oxide residues and preferably 4 to 20 ethylene oxide residues; polyoxyethylenated castor oil containing 10 to 60 moles of ethylene oxide; propylene oxide/ethylene oxide copolymers; soybean or egg yolk lecithin; sucroglycerides; soaps; fatty alcohol phosphoric esters; fatty alcohol sulphates optionally oxyethylenated; lanolin alcohols, polyoxyethylenated and comprising at least 4 ethylene oxide residues.

10. Cosmetic screening composition according to any one of Claims 1 to 9, characterised in that it contains in addition thickeners, emollients, humectants, surface-active agents, preservatives, antifoams, perfumes, dyes and pigments.

**11.** Cosmetic screening emulsion according to any one of Claims 1 to 10, characterised in that it is provided in the form of a milk or cream or is prepared as an aerosol.

**12.** Cosmetic screening emulsion according to any one of Claims 1 to 11, characterised in that it comprises in addition other water soluble or fat soluble UV screens chosen from among coffee oil, salicylic acid derivatives, cinnamic acid derivatives, p-aminobenzoic acid esters and derivatives, anthranilates, benzophenone derivatives, benzylidenecamphor derivatives such as p-methylbenzylidenecamphor, dibenzoylmethane derivatives, benzotriazole derivatives, benzoxazole derivatives, benzimidazole derivatives and alkyl ($C_2$-$C_{10}$) $\alpha$-cyano-$\beta$,$\beta$-diphenylacrylates.

**13.** Process for cosmetic treatment of the human epidermis with a view to protecting it against ultraviolet radiation of wavelengths between 280 and 400 nm, characterised in that it consists in applying to the skin an efficacious quantity of a cosmetic screening emulsion according to any one of Claims 1 to 12.

**14.** Process for preparing a cosmetic emulsion for screening out UV rays of wavelengths between 280 and 400 nm, characterised in that it consists in incorporating into the aqueous phase of the emulsion capable of containing lower monoalcohols or polyalcohols containing 1 to 6 carbon atoms, 0.1 to 10% by weight relative to the total weight of the composition, benzene-1,4-[di(3-methylidenecamphormethylsulphonic)] acid, of formula (I) according to Claim 1, partially or completely neutralised, in incorporating into the oily phase of the emulsion, consisting of fatty substances chosen from mineral oils, modified or non-modified vegetable or animal oils, saturated or unsaturated fatty acid esters, synthetic oils, waxes, silicone oils, $C_{12}$-$C_{18}$ fatty acids and fatty alcohols, 0.1 to 10% by weight of 2,4,6-tris[p-(2'-ethylhexyl-1'-oxycarbonyl)anilino]-1,3,5-triazine, and in adding emulsifiers and optionally at least one cosmetic adjuvant chosen from thickeners, emollients, humectants, surface-active agents, preservatives, antifoams, perfumes, dyes, pigments and other UV-screening agents.